# EUROPEAN PATENT SPECIFICATION

(11) **EP 3 387 987 B1**
(45) Date of publication and mention of the grant of the patent: **03.08.2022**
(21) Application number: 18167155.3
(22) Date of filing: 13.04.2018
(51) Int. Cl.: A61B 5/00, A61B 5/0537, A61B 5/11

(54) **AN APPARATUS FOR MEASURING BIOELECTRICAL IMPEDANCE OF A PATIENT**
APPARAT ZUR MESSUNG DER BIOELEKTRISCHEN IMPEDANZ EINES PATIENTEN
APPAREIL DE MESURE DE L'IMPÉDANCE BIOÉLECTRIQUE D'UN PATIENT

(30) Priority: 13.04.2017 IT 201700041695
(43) Date of publication of application: 17.10.2018
(73) Proprietor: BOCAhealthcare GmbH, 10405 Berlin (DE); Baiocco, Pietro, 00154 Roma (IT)
(72) Inventor: FARAGLI, Alessandro, 15121 Alessandria (AL) - Italy (IT); LA PORTA, Edoardo, 27100 Pavia (PV) - Italy (IT); ESTIENNE, Lorenzo, 10135 Torino (TO) - Italy (IT); BAIOCCO, Pietro, 00154 Roma (IT)
(74) Representative: Rapisardi, Mariacristina

(56) References cited:
- WO-A1-2011/075767
- US-A1- 2005 234 308
- US-A1- 2015 272 501
- US-A1- 2015 374 256

## Description

The present invention refers to an apparatus for measuring bioelectrical impedance of a patient.

Bioelectrical impedance analysis (BIA) is a non-invasive method that is simple to use and relatively inexpensive. It requires the positioning of electrodes on the body surface. One of the advantages of this method lies in its simplicity. The method comprises sending a low-voltage current pulse through the body which is not detectable by the patient (about 800 mA at a frequency of 5-50 kHz) and measuring the resistance of the body to the pulse. The underlying theory is that a different body composition influences resistance to the electric current. For example, lean body mass offers less resistance to the current because it contains more water and electrolytes compared to adipose tissue. Another advantage offered by this procedure is that it does not require special training or particular skills. Two types of devices are used for bioelectrical impedance analysis in clinical practice. The external impedance analyser is the most widely used and it provides the positioning of 2 pairs of electrodes (between the neck and the chest or between the wrist and the ankle). At least four electrodes are needed to obtain a reliable measurement.

An additional procedure for bioelectrical impedance analysis is carried out by means of implantable devices such as pacemakers and/or defibrillators, which analyse a current generated from the catheters of these same devices. The analysis concerns the thoracic compartment only.

Bioelectrical impedance analysis can be influenced by various factors such as blood volume, the composition of fluids, the positioning of the patient, the positioning of the electrodes, possible movements, skin sweat, blood composition (e.g. haematocrit) and body composition (e.g. the amount of fat).

Owing to all of the confounding factors cited above, the single measurement of a patient's fluid status does not prove to be very reliable and this represents the main drawback of this method. However, measurement of the change in hydration status (the delta value with respect to the baseline measurement) proves to be sensitive and thus useful clinically.

In the field of cardiology, its main application is in monitoring haemodynamics and the increase in extracellular volume in patients with chronic heart failure. By means of the cardiac cycle, implantable impedance analysers calculate cardiac output, the ejection fraction and volume overload. The use of these devices is common in clinical practice although continuous remote monitoring by these devices is not particularly widespread. Moreover, only a small part of the population affected by chronic heart failure has implanted devices given their invasiveness.

In the field of nephrology, bioelectrical impedance analysis has two main applications: for patients with chronic kidney failure undergoing conservative treatment and patients with terminal chronic kidney failure undergoing dialysis. Its main use concerns monitoring the nutritional status of patients who develop muscle cachexia and monitoring liquids in patients with nephrotic syndrome, chronic kidney failure and heart failure with a concomitant reduction in diuresis. Measurement of a patient's actual hydration status can prove to be difficult even using different standardized techniques in addition to impedance analysis (ultrasound, central venous pressure, objective examination). At this time, one single exam capable of accurately defining the volume status of a patient does not exist. Various non-invasive and/or invasive exams and an evaluation of the overall results are needed to evaluate the actual extracellular volume of a patient. Advanced chronic kidney failure added to heart failure and nephrotic syndrome lead to the tendency to accumulate liquids and the need for significant diuretic treatment. This can lead to the opposite condition, that is, to the depletion of volume with resulting hypotension, acute kidney failure and even a state of shock. There are also numerous possible uses for other types of patients: obese patients, patients suffering from anorexia, patients in intensive care, patients in postoperative care, pregnant women, puerperae and patients affected by HIV. Moreover, the analysis of volume depletion can be particularly useful in the case of elderly patients confined to bed and/or residents of nursing homes with medical services and care.

The use of bioelectrical impedance analysis for measuring the nutritional status of healthy adults, sports players and athletes is widespread, especially for monitoring lean body mass and fat body mass. This is done by means of electrical impedance scales, which are not highly sensitive for determining the concentration of free body water, which is very rapidly influenced by gravity.

At present, only a very limited number of companies produce bioelectrical impedance analysers. Current bioelectrical impedance analysers do not usually offer the possibility of connection with smartphones or tablets. With the present devices, there is no possibility for the patient to take measurements at home without the assistance of another person. Essentially, at this time there is no bioelectrical impedance analyser on the market that is easy to use and that enables totally reliable and independent use by the patient.

A wearable bioimpedance analysis device is disclosed by US 2015/272501.

The technical task of the present invention is therefore to realize an apparatus for measuring the bioelectrical impedance of a patient which makes it possible to eliminate the cited technical drawbacks of the prior art.

Within the scope of this technical task, an aim of the invention is to realize an apparatus for measuring bioelectrical impedance of a patient, which is convenient and easy to use even without the assistance of external medical personnel.

Another aim of the invention is to realize an apparatus for measuring bioelectrical impedance of a patient, which is suitable for enabling constant monitoring of the patient by the physician.

The technical task, as well as the latter and other aims, according to the present invention, are achieved by realizing an apparatus for measuring bioelectrical impedance of a patient as defined in claim 1.

In a preferred embodiment of the invention, said first and second motion sensor comprise an accelerometer and/or a gyroscope.

According to the invention, said first and second connecting means are via electric cable.

In a preferred embodiment of the invention, said first and second band are elastic. In a preferred embodiment of the invention, said first and second band are loop-wound and have re-closable opening means.

According to the invention, said first band is made of a soft material and has a rigid insert housing said first pair of electrodes and said first motion sensor.

According to the invention, said second band is made of a soft material and has a rigid insert housing said second pair of electrodes and said second motion sensor.

According to the invention, said first connecting means comprise a first electric cable having a terminal connector removably connected to an electrical connector of said first band.

According to According to the invention, said second connecting means comprise a second electric cable having a terminal connector removably connected to an electrical connector of said second band.

In a preferred embodiment of the invention, said portable device has a button for switching on and activating the wireless connection with a remote electronic device, for example, a smartphone.

The apparatus in accordance with the invention is portable and easy to use, medically reliable and nears gold standard levels obtained in hospital settings, although it is useable at home, without the assistance of externa medical personnel. Advantageously, by means of a software application that is downloadable on a smartphone and compatible with all operating systems (iOS, Android, Windows Phone), continuous monitoring of a patient by a physician can be carried out and interaction and interface through a website.

Further characteristics and advantages of the invention will become more apparent from the description of a preferred, but not exclusive, embodiment of the apparatus for measuring bioelectrical impedance of a patient according to the invention, which is illustrated by way of indicative and non-limiting example in the attached drawings, of which:

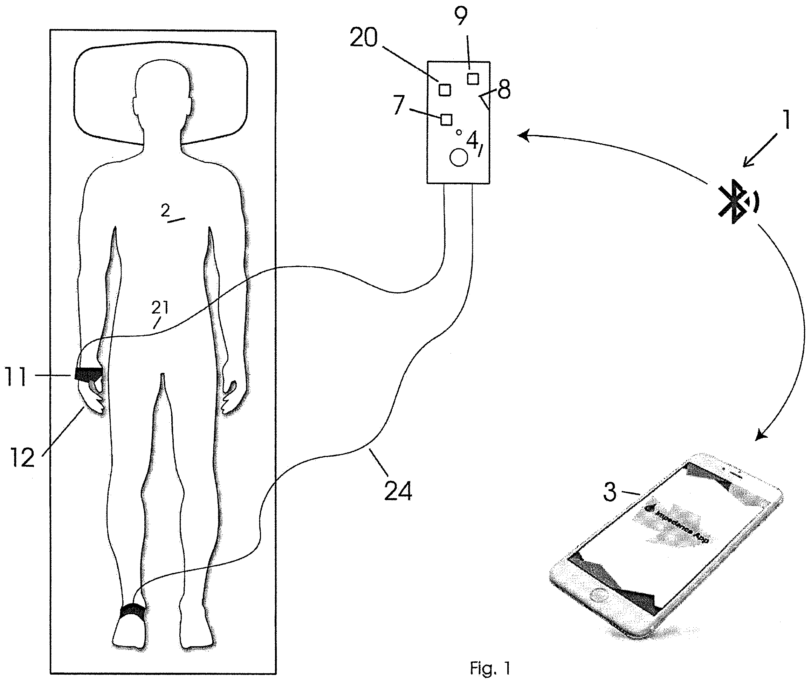

figure 1 schematically shows the apparatus in a first version without an abdominal belt;
figure 2 schematically shows the apparatus in a second version with an abdominal belt;

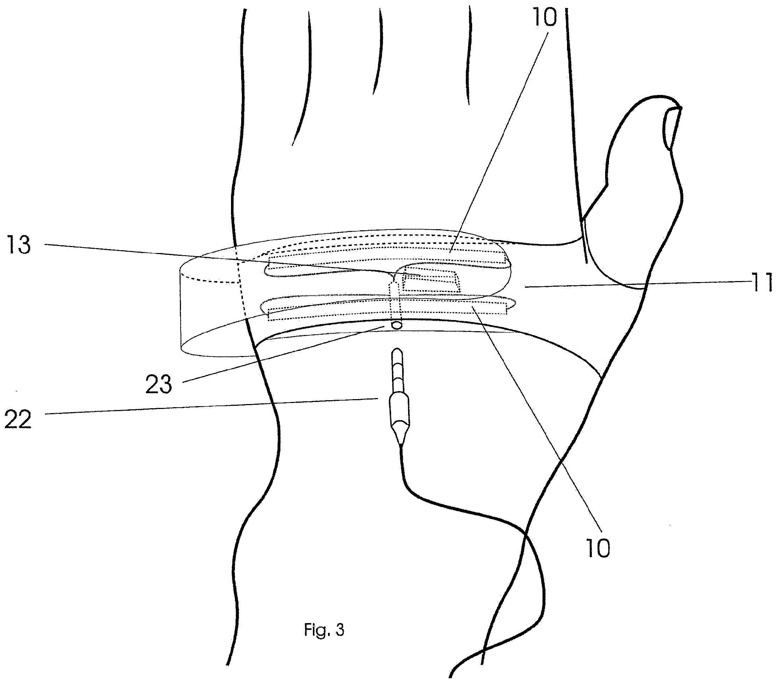

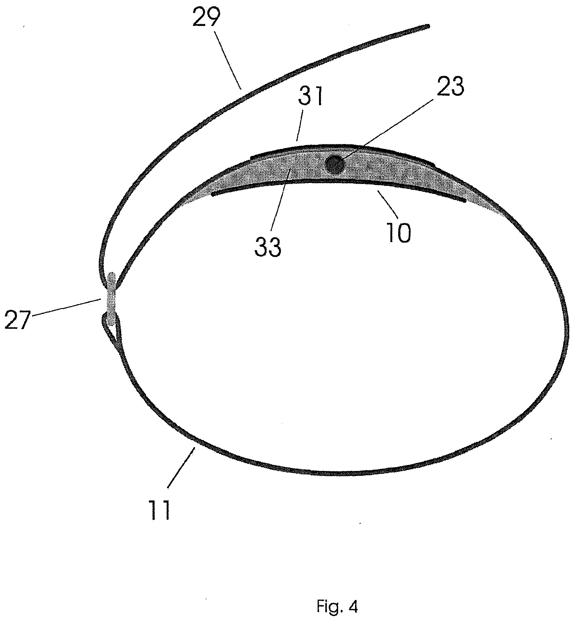

figures 3 and 4 schematically show the first band.

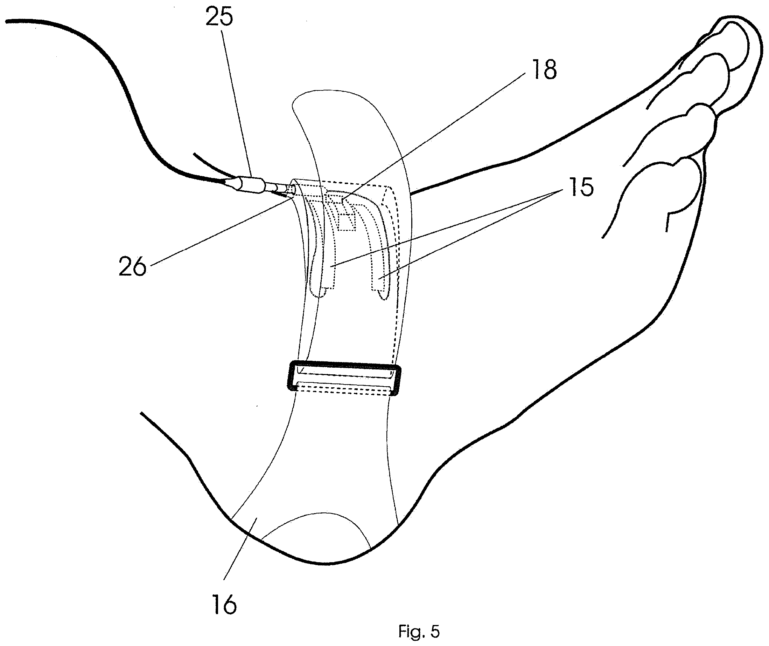

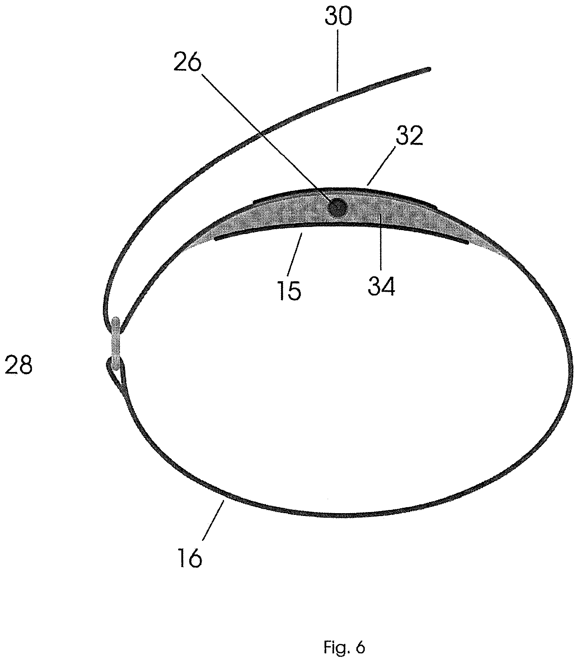

Figures 5 and 6 schematically show the second band.
Figure 7 is a schematic block diagram of the system according to an embodiment of the present invention: the configuration illustrated and described is a purely illustrative example of one type of implementation and therefore it does not limit the scope of the present invention; the arrangement and the shape of the components inside the central instrumental unit and inside the electrodes, for the hand and/or foot, are purely illustrative examples and should be exclusively understood as a working method for the various devices involved.

With reference to the figures cited, an apparatus for measuring bioelectrical impedance of a patient is shown and indicated in its entirety by the reference number 1.

The apparatus 1 comprises a portable device 4 for measuring bioelectrical impedance, a first pair of electrodes 10, a first band 11 having a conformation suitable for fitting on a hand 12 of the patient 2 and incorporating the first pair of electrodes 10 and at least a first motion sensor 13, first connecting means 14 for connecting the first pair of electrodes 10 with the portable device 4, a second pair of electrodes 15, a second band 16 having a conformation suitable for fitting on a foot 17 of the patient 2 and incorporating the second pair of electrodes 15 and at least a second motion sensor 18, and second connecting means 19 for connecting the second pair of electrodes 15 with the portable device 4.

The device 4 also has a button 40 for switching on and activating the wireless connection.

The portable device 4 comprises a box-like support 8 substantially shaped in the form of a parallelepiped and that supports a controller (not shown), for example an electronic board, a rechargeable electrical energy accumulator (not shown), for example an accumulator that can be recharged by a solar battery (not shown) and/or a USB port 9, a wireless data receiving and transmitting unit 7 suitable for communication with a remote electronic device, for example a smartphone 3 or a tablet, so as to transmit at least the measurement data.

The wireless data receiving and transmitting unit 7 communicates with a software application installed on the remote electronic device preferably via Bluetooth technology.

The first motion sensor 13 and the second motion sensor 18 comprise an accelerometer and/or a gyroscope.

The first connecting means 14 and the second connecting means 19 are via electric cable.

The first connecting means 14 comprise a first electric cable 21 having a terminal connector 22 removably connected to an electrical connector 23 of the first band 11.

The second connecting means 19 comprise a second electric cable 24 having a terminal connector 25 removably connected to an electrical connector 26 of the second band 16.

Each band 11, 16 is elastic and specifically made of a soft material, for example a stretch fabric, and has a rigid insert 33, 34 housing the electrodes 10, 15 and the motion sensor 13, 18.

The rigid insert 33, 34 has an elongated box-like conformation and is applied to a longitudinal section of the band 11, 16.

The insert 33, 34 has an exposed base wall on which the provided electrodes 10, 15 in the form of longitudinal strips are positioned, and an exposed lateral wall, on which the electrical connector 23, 26 is positioned, whereas the motion sensor 13, 18 is positioned inside the insert 33, 34.

Each band 11, 16 is also loop-wound and has re-closable opening means.

The re-closable opening means are, for example, of a velcro type and thus comprise a grommet 27, 28, in which one end edge 29, 30 of the band 11, 16 is inserted, and a fabric hook-and-loop closure system, known under the brand name of Velcro^{™}, between the end edge 29, 30 of the band 11, 16 and an edge 31, 32 of the band 11, 16, which, with respect to the grommet 27, 28, is located opposite the end edge 29, 30.

In one embodiment of the first connecting means 14 which are necessary for sending the acquired data and/or for receiving the pulse from the portable device 4, the electrical connector 22 is a jack plug and the electrical connector 23 is a jack socket, which, if the first band 11 is applied correctly in the position illustrated, is perpendicular to the dorsal side of the hand and in the direction of the wrist. The electrical cable 21 at the opposite end can be connected permanently to the portable device 4 or once again by means of a removable jack plug/jack socket connection.

The first connecting means 14 preferably also connect the first motion sensor 13 to the portable device 4 for transmitting the data detected and for supplying power. It can also be comprised that the first motion sensor 13 can communicate directly with the remote electronic device by means of a wireless transmission channel, for example an IR transmission channel.

In one embodiment of the second connecting means 19, which are needed for sending the acquired data and/or for receiving the pulse from the portable device 4, the electrical connector 25 is a jack plug and the electrical connector 26 is a jack socket, which, if the second band 16 is applied correctly in the position illustrated, is perpendicular to the instep of the foot and in the direction of the tibia. The electrical cable 24 at the opposite end can be connected permanently to the portable device 4 or once again by means of a removable jack plug/jack socket connection.

The second connecting means 19 preferably also connect the second motion sensor 18 to the portable device 4 for transmitting the data detected and for supplying power. It can also be comprised that the second motion sensor 18 can communicate directly with the remote electronic device by means of a wireless transmission channel, for example an IR transmission channel.

The motion sensors 13, 10 cooperate to identify the position of the patient 2.

In particular, two accelerometers can be used, one installed in the first band 11 and one installed in the second band 16, each having a calculation board (PCB with a microcontroller) connected to an infrared-ray antenna.

The accelerometer that can be employed to detect and/or measure acceleration on the axes X, Y and Z is part of the family of MEMS (Micro Electro-Mechanical Systems). Normally the electronics and mechanical parts of these devices are contained in a package of dimensions equal to or smaller than 3x3xlmm. Among the many applications that these devices offer, there is also the possibility of obtaining a value that refers to a position, and this function is used to discern whether or not the patient is positioned in an appropriate position for obtaining a reliable measurement.

For example, the appropriate position is a horizontal position assumed when the patient is lying down on a bed and rests his/her foot and hand (on which the bands 11, 16 are applied) on the bed.

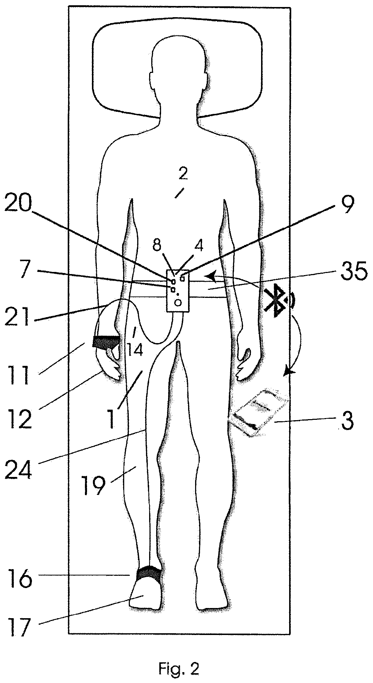

In the solution shown in Figure 2, the portable device 4 is integrated, preferably removably, in an adjustable abdominal belt 35 that is wearable by the patient 2.

The abdominal belt 35 particularly provides a snap-fit mechanism that enables the portable device 4 to be positioned over the patient's abdomen. The advantage of this solution is that the patient can take continuous measurements even throughout the rest of the day.

In other, unillustrated solutions, the first band 11 can be removably associated with a glove, whereas the second band 16 can be removably associated with a sock. The operation of the apparatus 1 is as follows.

The measuring process is started when the motion sensors 13, 18 signal the continuance of the correct measurement position for a predetermined period of time.

The total time needed to take an adequate measurement is established as equal to two minutes for example. The first minute is needed for stabilization of the patient in the horizontal position, whereas the second minute is needed for the actual measurement of impedance.

If the patient moves from the horizontal position to the vertical position in the first two minutes, the measurement is taken, but a notification that the measurement has not been taken correctly and should be repeated will appear on the smartphone 3 or the measurement is acquired all the same with a specific note.

In conclusion, the motion sensors must detect the correct position for a period of 2 minutes in order to obtain an adequate measurement.

The presence of the motion sensors to identify the correct position of the patient ensures punctual or continuous measurements that are repeatable and accurate. The measurement results (taken correctly or not) shall be sent to the referring physician who will be able to understand the degree of accuracy of the measurement.

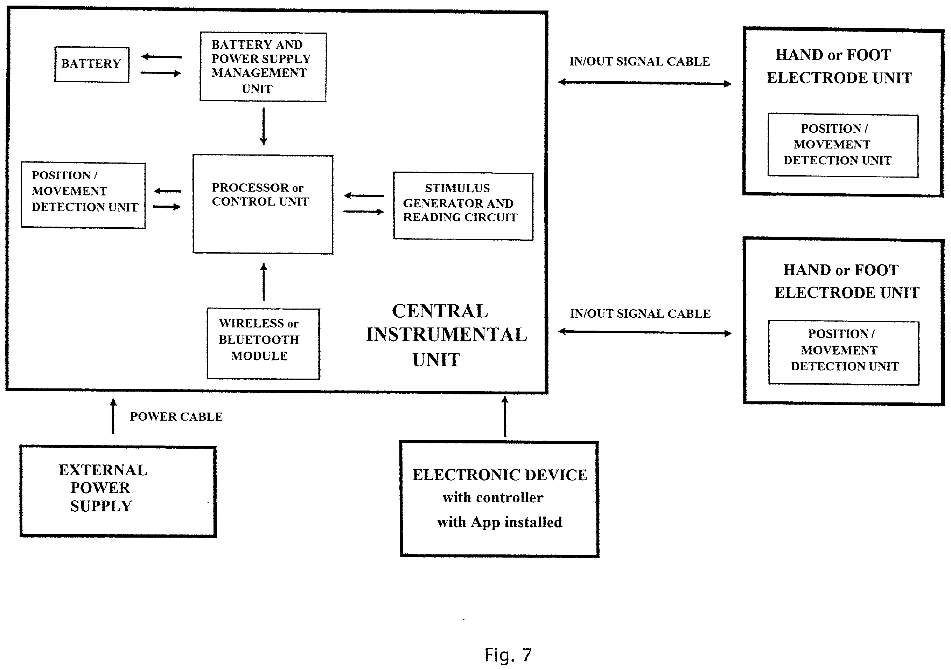

As illustrated in Figure 7, in another embodiment of the invention, the system includes a central instrumental unit, which in one particular case can be the portable device cited hereinabove, and two or more electrode units equipped with one or more sensors.

The central instrumental unit is required to implement the functions which include for example: controlling the generator of stimuli and the reading circuit, which is the dedicated subsystem for impedance measurement; the interface with an App by means of wireless communication, which for example can be Bluetooth; and the interface with one or more of the sensors for detecting the environmental conditions of the electrode units, for example position and movement.

Each electrode unit includes all the electrodes needed for the measurements and one or more sensors for detecting the environmental conditions of the terminal. The control and processing functions of the terminal signal are distributed among these devices based on their respective functions.

The system includes a management unit for managing the supply of energy that powers all the subsystems and it can be battery-powered.

The system is interfaced by means of the app, which can be installed on any electronic device and it can enable setting of the parameters, sending commands to the control unit, receiving or sending data and information, for example about the system.

Bioelectrical impedance measurement is a very useful tool for the evaluation of the trend of the water balance of the body. An absolute measurement is not particularly useful and it may not be reliable.

However, a change in hydration status over a period of time can be extremely important clinically and diagnostically and it is definitely much more reliable. The possibility of rapid and repeatable bioelectrical impedance measurement in both the hospital and the home setting can represent a significant improvement in the quality of health and life of the chronic patient. Its use would improve the diagnostic process and the ability to carry out remote monitoring of patients, reducing acute episodes, complications and hospital readmissions.

The apparatus in accordance with the invention makes it possible to improve the accuracy of diagnoses especially for detection of acute and chronic disorders of extracellular volume (retention or depletion) in patients affected by chronic cardiovascular and nephrotic diseases.

The apparatus in accordance with the invention provides for a continuous physician-patient interface. The application allows for a dual interface: patient and physician. The physician can see both interfaces and especially the graphics needed to interpret the measurements obtained from the patient over a period of time. As regards the patient interface, the software developed makes it possible to take measurements correctly with both visual and voice instructions. The principal aim of the application is to enable the physician to monitor the patient's status remotely. Vice versa, the patient can be in contact with the physician at all times or send data by means of a fast connection with the hospital or with the outpatient facility in charge of his/her care.

The apparatus in accordance with the invention contributes to improving the patient's quality of life. In fact, the patient can send data on his/her water retention state even in situations in which there is no immediate access to medical care. With the organization of a calendar, the physician will be able to provide for the best diuretic treatment by analysing the previous water balance and the dosage of diuretic administered and intervene on the imbalance emerging in the patient's clinical picture. The software also provides for a coaching program aimed at correct administration of the diuretic, a proper diet and proper intake of water. Furthermore, for athletes and sports players, a special graphical interface and specific hardware will be developed subsequently, taking their different needs into account.

The software application can be downloaded on any smartphone or tablet and it is compatible with the iOS, Android and Windows Phone operating systems.

The interaction between the hardware and software makes it possible to monitor the following values: the water balance (overhydration versus dehydration); total water content (intracellular and extracellular); lean body mass; fat body mass; and body cellular mass.

Additionally, the interaction between the hardware and software can integrate the following functions: the remote connection with the clinic in charge of the patient via email; the patient's profile, a fitness organizer and a coaching program for the patient; and the patient's position (horizontal vs. vertical).

These functions can be expanded and as an example, electrocardiogram (ECG) analysis can be implemented by using the electrodes that are already present.

The apparatus for measuring bioelectrical impedance thus conceived is susceptible to numerous modifications and variants, all of which falling within the scope of the inventive concept. Moreover, all details may be replaced with other technically equivalent elements.

The materials used, as well as the dimensions, may in practice be of any type, according to needs and the state of the art.

## Claims

1. An apparatus (1) for measuring bioelectrical impedance of a patient (2), comprising : a portable device (4) for measuring bioelectrical impedance provided with a controller, an electrical energy accumulator and a wireless data receiving and transmitting unit (7), a first pair of electrodes (10) and a second pair of electrodes (15), a first band (11) having a conformation suitable for fitting on a hand (12) of a patient (2) and incorporating said first pair of electrodes (10) and at least a first motion sensor (13), a second band (16) having a conformation suitable for fitting on a foot (17) of the patient (2) and incorporating said second pair of electrodes (15) and at least a second motion sensor (18), first connecting means (14) of said first pair of electrodes (10) with said portable device (4), and second connecting means (19) of said second pair of electrodes (15) with said portable device (4), said first connecting means (14) comprising a first electric cable (21) having a terminal connector (22) removably connected to an electrical connector (23) of said first band (11), said second connecting means (19) comprising a second electric cable (24) having a terminal connector (25) removably connected to an electrical connector (26) of said second band (16), said first band (11) is made of a soft material and has a rigid insert (33) housing said first pair of electrodes (10) and said first motion sensor (13), said second band (16) is made of a soft material and has a rigid insert (34) housing said second pair of electrodes (15) and second motion sensor (18), each rigid insert (33, 34) having an elongated box-like conformation and being applied to a longitudinal section of the respective band (11, 16), each insert (33, 34) having an exposed base wall on which the respective provided electrodes (10, 15) in the form of longitudinal strips are positioned, and an exposed lateral wall, on which the respective electrical connector (23, 26) is positioned.

2. The apparatus (1) for measuring bioelectrical impedance according to claim 1, **characterised in that** said first and second motion sensor (13, 18) comprise an accelerometer and/or a gyroscope.

3. The apparatus (1) for measuring bioelectrical impedance according to claim 1, **characterised in that** said first and second connecting means (14, 19) are via electric cable.

4. The apparatus (1) for measuring bioelectrical impedance according to claim 1, **characterised in that** said first and second band (11, 16) are elastic.

5. The apparatus (1) for measuring bioelectrical impedance according to claim 1, **characterised in that** said first and second band (11, 16) are loop-wound and have re-closable opening means.

6. The apparatus (1) for measuring bioelectrical impedance according to the preceding claim, **characterised in that** said opening means are of a velcro type.

7. The apparatus (1) for measuring bioelectrical impedance according to claim 1, **characterised in that** said portable device (4) is integrated in an adjustable abdominal belt (35) wearable by the patient (2).

8. The apparatus for measuring bioelectrical impedance of a patient according to claim 1, **characterised in that** said portable device (4) has a button (19) for switching-on and activating the wireless connection.

9. The apparatus for measuring bioelectrical impedance of a patient according to claim 1, **characterised in that** said first band (11) is removably associated to a glove.

10. The apparatus for measuring bioelectrical impedance of a patient according to claim 1, **characterised in that** said second band (16) is removably associated to a sock.

## Patentansprüche

1. Apparat (1) zur Messung der bioelektrischen Impedanz eines Patienten (2), umfassend: eine tragbare Vorrichtung (4) zur Messung der bioelektrischen Impedanz, die mit einem Controller, einem elektrischen Energiespeicher und einer drahtlosen Datenempfangs- und -übertragungseinheit (7), einem ersten Paar von Elektroden (10) und einem zweiten Paar von Elektroden (15) versehen ist, wobei ein erstes Band (11) eine Gestalt aufweist, die zum Anbringen an einer Hand (12) eines Patienten (2) geeignet ist und das erste Paar von Elektroden (10) und mindestens einen ersten Bewegungssensor (13) enthält, wobei ein zweites Band (16) eine Gestalt aufweist, die zum Anbringen an einem Fuß (17) des Patienten (2) geeignet ist und das zweite Paar von Elektroden (15) und mindestens einen zweiten Bewegungssensor (18), erste Verbindungsmittel (14) des ersten Paars von Elektroden (10) mit der tragbaren Vorrichtung (4) und zweite Verbindungsmittel (19) des zweiten Paars von Elektroden (15) mit der tragbaren Vorrichtung (4) enthält, wobei die ersten Verbindungsmittel (14) ein erstes elektrisches Kabel (21) umfassen, das einen Anschlussverbinder (22) aufweist, der entfernbar mit einem elektrischen Verbinder (23) des ersten Bandes (11) verbunden ist, wobei die zweiten Verbindungsmittel (19) ein zweites elektrisches Kabel (24) umfassen, das einen Anschlussverbinder (25) aufweist, der entfernbar mit einem elektrischen Verbinder (26) des zweiten Bandes (16) verbunden ist, wobei das erste Band (11) aus einem weichen Material hergestellt ist und einen starren Einsatz (33) aufweist, der das erste Paar von Elektroden (10) und den ersten Bewegungssensor (13) aufnimmt, wobei das zweite Band (16) aus einem weichen Material hergestellt ist und einen starren Einsatz (34) aufweist, der das zweite Paar von Elektroden (15) und den zweiten Bewegungssensor (18) aufnimmt, wobei ein jeder starre Einsatz (33, 34) eine längliche kastenartige Gestalt aufweist und an einem Längsabschnitt des jeweiligen Bandes (11, 16) angebracht ist, wobei ein jeder Einsatz (33, 34) eine freiliegende Basiswand, an der die jeweiligen vorgesehenen Elektroden (10, 15) in Form von Längsstreifen positioniert sind, und eine freiliegende Seitenwand aufweist, an der der jeweilige elektrische Verbinder (23, 26) positioniert ist.

2. Apparat (1) zur Messung der bioelektrischen Impedanz nach Anspruch 1, **dadurch gekennzeichnet, dass** der erste und der zweite Bewegungssensor (13, 18) einen Beschleunigungsmesser und/oder ein Gyroskop umfassen.

3. Apparat (1) zur Messung der bioelektrischen Impedanz nach Anspruch 1, **dadurch gekennzeichnet, dass** die ersten und die zweiten Verbindungsmittel (14, 19) über ein elektrisches Kabel ausgestaltet sind.

4. Apparat (1) zur Messung der bioelektrischen Impedanz nach Anspruch 1, **dadurch gekennzeichnet, dass** das erste und das zweite Band (11, 16) elastisch sind.

5. Apparat (1) zur Messung der bioelektrischen Impedanz nach Anspruch 1, **dadurch gekennzeichnet, dass** das erste und das zweite Band (11, 16) in Schlaufen gewickelt sind und wiederverschließbare Öffnungsmittel aufweisen.

6. Apparat (1) zur Messung der bioelektrischen Impedanz nach dem vorhergehenden Anspruch, **dadurch gekennzeichnet, dass** die Öffnungsmittel vom Klett-Typ sind.

7. Apparat (1) zur Messung der bioelektrischen Impedanz nach Anspruch 1, **dadurch gekennzeichnet, dass** die tragbare Vorrichtung (4) in einen einstellbaren Bauchgurt (35) integriert ist, der vom Patienten (2) getragen werden kann.

8. Apparat zur Messung der bioelektrischen Impedanz eines Patienten nach Anspruch 1, **dadurch gekennzeichnet, dass** die tragbare Vorrichtung (4) einen Knopf (19) zum Einschalten und Aktivieren der drahtlosen Verbindung aufweist.

9. Apparat zur Messung der bioelektrischen Impedanz eines Patienten nach Anspruch 1, **dadurch gekennzeichnet, dass** das erste Band (11) entfernbar mit einem Handschuh assoziiert ist.

10. Apparat zur Messung der bioelektrischen Impedanz eines Patienten nach Anspruch 1, **dadurch gekennzeichnet, dass** das zweite Band (16) entfernbar mit einer Socke verbunden ist.

## Revendications

1. Appareil (1) de mesure de l'impédance bioélectrique d'un patient (2), comprenant : un dispositif portable (4) de mesure de l'impédance bioélectrique pourvu d'un contrôleur, d'un accumulateur d'énergie électrique et d'une unité de réception et de transmission (7) de données sans fil, une première paire d'électrodes (10) et une deuxième paire d'électrodes (15), une première bande (11) ayant une conformation appropriée pour s'adapter sur une main (12) d'un patient (2) et incorporant ladite première paire d'électrodes (10) et au moins un premier capteur de mouvement (13), une deuxième bande (16) ayant une conformation appropriée pour s'adapter sur un pied (17) du patient (2) et incorporant ladite deuxième paire d'électrodes (15) et au moins un deuxième capteur de mouvement (18), des premiers moyens de raccordement (14) de ladite première paire d'électrodes (10) audit dispositif portable (4), et des deuxièmes moyens de raccordement (19) de ladite deuxième paire d'électrodes (15) audit dispositif portable (4), lesdits premiers moyens de raccordement (14) comprenant un premier câble électrique (21) comportant un connecteur terminal (22) connecté de manière amovible à un connecteur électrique (23) de ladite première bande (11), lesdits deuxièmes moyens de raccordement (19) comprenant un deuxième câble électrique (24) ayant un connecteur terminal (25) connecté de manière amovible à un connecteur électrique (26) de ladite deuxième bande (16), ladite première bande (11) est constituée d'un matériau souple et comporte un insert rigide (33) logeant ladite première paire d'électrodes (10) et ledit premier capteur de mouvement (13), ladite deuxième bande (16) est constituée d'un matériau souple et comporte un insert rigide (34) logeant ladite deuxième paire d'électrodes (15) et le deuxième capteur de mouvement (18), chaque insert rigide (33, 34) ayant une conformation allongée en forme de boîte et étant appliqué à une section longitudinale de la bande respective (11, 16), chaque insert (33, 34) comportant une paroi de base exposée sur laquelle sont positionnées les électrodes prévues (10, 15) respectives sous la forme de rubans longitudinaux, et une paroi latérale exposée, sur laquelle est positionné le connecteur électrique (23, 26) respectif.

2. Appareil (1) de mesure de l'impédance bioélectrique selon la revendication 1, **caractérisé en ce que** lesdits premier et deuxième capteurs de mouvement (13, 18) comprennent un accéléromètre et/ou un gyroscope.

3. Appareil (1) de mesure de l'impédance bioélectrique selon la revendication 1, **caractérisé en ce que** lesdits premier et deuxième moyens de raccordement (14, 19) le sont par câble électrique.

4. Appareil (1) de mesure de l'impédance bioélectrique selon la revendication 1, **caractérisé en ce que** lesdites première et deuxième bandes (11, 16) sont élastiques.

5. Appareil (1) de mesure de l'impédance bioélectrique selon la revendication 1, **caractérisé en ce que** lesdites première et deuxième bandes (11, 16) sont enroulées en boucle et comportent des moyens d'ouverture refermables.

6. Appareil (1) de mesure de l'impédance bioélectrique selon la revendication précédente, **caractérisé en ce que** lesdits moyens d'ouverture sont de type à velcro.

7. Appareil (1) de mesure de l'impédance bioélectrique selon la revendication 1, **caractérisé en ce que** ledit dispositif portable (4) est intégré dans une ceinture abdominale réglable (35) pouvant être portée par le patient (2).

8. Appareil de mesure de l'impédance bioélectrique d'un patient selon la revendication 1, **caractérisé en ce que** ledit dispositif portable (4) comporte un bouton (19) de mise en marche et d'activation de la connexion sans fil.

9. Appareil de mesure de l'impédance bioélectrique d'un patient selon la revendication 1, **caractérisé en ce que** ladite première bande (11) est associée de manière amovible à un gant.

10. Appareil de mesure de l'impédance bioélectrique d'un patient selon la revendication 1, **caractérisé en ce que** ladite deuxième bande (16) est associée de manière amovible à une chaussette.
